# EUROPÄISCHE PATENTSCHRIFT

(11) **EP 2 942 071 B1**
(45) Veröffentlichungstag und Bekanntmachung des Hinweises auf die Patenterteilung: **23.11.2016**
(21) Anmeldenummer: 15174718.5
(22) Anmeldetag: 16.07.2014
(51) Int. Cl.: A61M 1/16

(54) **EXTRAKORPORALE BLUTBEHANDLUNGSANLAGE MIT WÄRMERÜCKGEWINNUNG**
EXTRACORPOREAL BLOOD PROCESSING DEVICE WITH HEAT RECOVERY
INSTALLATION DE TRAITEMENT DU SANG EXTRA-CORPOREL DOTÉE D'UNE RÉCUPÉRATION DE CHALEUR

(30) Priorität: 18.07.2013 DE 102013107672
(43) Veröffentlichungstag der Anmeldung: 11.11.2015
(62) Teilanmeldung aus: 14177206.1
(73) Patentinhaber: B. Braun Avitum AG, 34212 Melsungen (DE)
(72) Erfinder: Rohde, Alexander, 34212 Melsungen (DE); Rott, Siegmar, 34131 Kassel (DE); Bröker, Björn, 34355 Staufenberg (DE)
(74) Vertreter: Winter, Brandl, Fürniss, Hübner, Röss, Kaiser, Polte - Partnerschaft mbB

(56) Entgegenhaltungen:
- US-A- 4 804 474

## Beschreibung

Die vorliegende Erfindung betrifft eine extrakorporale Blutbehandlungsanlage, insbesondere Dialyseanlage, mit Wärmerückgewinnung sowie zentralem Energiemanagement und des Weiteren eine extrakorporale Blutbehandlungsanlage, insbesondere Dialyseanlage, bestehend aus einer stationären Wasser-/Fluidversorgung sowie selektiv daran anschließbaren Blutbehandlungsmaschinen (Dialysemaschinen) vorzugsweise der mobilen Bauart gemäß dem Oberbegriff des Patentanspruchs 1.

### Hintergrund der Erfindung

Extrakorporale Blutbehandlungsanlagen, vorzugsweise Dialyseanlagen der einschlägigen Gattung bestehen allgemein aus einer stationären Wasserversorgung mit einer Anzahl von Wasser-Anschlussmöglichkeiten (Zapfstellen), an die selektiv vorzugsweise mobile Blutbehandlungs-/Dialysemaschinen (Maschinen für Akutdialyse, Maschinen für Apherese, usw.) fluidtechnisch angeschlossen werden können. Derartige Blutbehandlungs-/Dialysemaschinen sind u. a. mit internen Heizsystemen ausgerüstet, um das aus der stationären Wasserversorgung abgezapfte Wasser in der Blutbehandlungs-/Dialysephase auf Körpertemperatur und in der Desinfektionsphase auf etwas unter Siedetemperatur (beispielsweise ca. 70°C - 90°C) aufzuheizen. Die hierfür erforderliche Primärenergie wird den Blutbehandlungs-/Dialysemaschinen in Form von elektrischem Strom bereitgestellt. Zusätzlich können die Blutbehandlungs-/Dialysemaschinen jeweils mit integrierten Wärmetauschern versehen sein, um Wärmeenergie in der verbrauchten Reinigungs-/Dialysierflüssigkeit dem aus der stationären Wasserversorgung abgezapften Wasser oder der daraus frisch aufbereiteten Reinigungs-/Dialysierflüssigkeit zuzuführen und dadurch den Primärenergieverbrauch der einzelnen Blutbehandlungs-/Dialysemaschinen zu senken.

Diese bekannten Konzepte beruhen folglich im Wesentlichen auf dem Prinzip der sogenannten Insellösung in Form von völlig unabhängig arbeitenden Blutbehandlungs-/Dialysemaschinen vor, die jeweils für sich (individuell) die erforderlichen Funktionsstoffe wie Desinfektionsmittel oder die Reinigungs-/Dialysierflüssigkeit, etc. in jeweils geeignetem Zustand (Temperatur, Konzentration) sowie zu jeweils geeigneten Zeitpunkten zur Verfügung stellen.

### Stand der Technik

Wie vorstehend bereits angedeutet wurde, muss bei einer extrakorporalen Blutbehandlung die durch den Reinigungsfilter fließende Reinigungsflüssigkeit auf eine voreingestellte Zieltemperatur - in der Regel ca. 37°C - erwärmt werden. In einer Dialysemaschine wird hierbei die einfließende Dialysierflüssigkeit/Wasser zunächst durch den vorstehend genannten internen Wärmetauscher vorgewärmt und anschließend durch eine Heizung auf den Zielwert vorzugsweise elektrisch aufgeheizt. Jedoch ist die Ausrüstung jeder Blutbehandlungs-/Dialysemaschine mit einem Wärmetauscher aufwändig und auch teuer. Je nach Anzahl von angeschlossenen Blutbehandlungs-/Dialysemaschinen wird daher die gesamte Blutbehandlungsanlage zunehmend kostenintensiv. Ein Verzicht auf den internen Wärmetauscher würde indessen die Betriebskosten aufgrund eines steigenden Primärenergieverbrauchs übergebührlich erhöhen.

In der WO 2012/175210 A2 ist ein Energieversorgungskonzept vorgeschlagen worden, das prinzipiell die Bereitstellung preisgünstig erzeugter Primärenergie vorsieht, welche beispielsweise in einem Dialysezentrum verbraucht wird, das mit einer Dialyseanlage gemäß vorstehendem Aufbau ausgestattet ist. Demnach ist u. a. eine Fotovoltaikanlage zur Erzeugung von elektrischem Strom mit einer Wärmepumpe insbesondere für das Erwärmen von Behandlungsräumen gepaart, welche hierfür thermische Energie aus einer Ablaufleitung für verbrauchte Dialysierflüssigkeit aufnimmt und Nutzenergie in die Behandlungsräume überträgt. Durch die Wärmepumpe kann somit die in der verbrauchten Dialysierflüssigkeit enthaltene Energie recycelt werden. Indessen bleibt das eingangs beschriebene Prinzip der Insellösung bezüglich der einzelnen Dialysemaschinen unberührt.

US 4804474 offenbart eine extrakorporale Blutbehandlungsanlage mit Wärmerückgewinnung.

### Kurzbeschreibung der Erfindung

Der vorliegenden Erfindung liegt angesichts dieses Stands der Technik die Aufgabe zugrunde, ein Energieversorgungskonzept für ein extrakorporales Blutbehandlungszentrum (wie Dialysezentrum) bereit zu stellen, mittels dem die laufenden Betriebskosten des extrakorporalen Blutbehandlungszentrums insgesamt verringert werden können.

Diese Aufgabe wird durch eine Blutbehandlungsanlage (wie Dialyseanlage) mit den Merkmalen des Anspruchs 1 oder 2 gelöst. Vorteilhafte Weiterbildungen der Erfindung sind Gegenstand der Unteransprüche.

Der vorliegenden Erfindung liegen die nachfolgenden energetischen Überlegungen zugrunde:
Die Verbesserung der Betriebseffizienz eines Blutbehandlungszentrums (Dialysezentrums) beschränkt sich nicht nur auf die Bereitstellung preisgünstig erzeugter Primärenergie und das Recycling bereits eingesetzter Energie mittels bekannter Energieumwandlungsanlagen sondern erstreckt sich auch auf deren vorteilhaften Einsatz. Dazu ist es erforderlich, die monetären Aufwendungen in einem Blutbehandlungszentrum (Dialysezentrum) für Beschaffung, Wartung und laufenden Betrieb zu analysieren und durch geeignetes Zentralisieren der eingesetzten Energien zu optimieren.

Es besteht beispielsweise die Möglichkeit, dass insbesondere bei Blutbehandlungs-/Dialysemaschinen deren individuelle Ausrüstung mit Wärmetauschern ggf. zu Kostensteigerungen insbesondere für Beschaffung und Wartung (einschließlich Desinfektion des Wärmetauschers) führt. Es ist zwar bekannt, dass parallel hierzu laufende Betriebskosten etwa für die Beheizung des Dialysezentrums beispielsweise durch alternative Energieumwandlungsanlagen gemäß dem genannten Stand der Technik reduzierbar sind. Jedoch stellt sich an dieser Stelle erfindungsgemäß die Frage, ob die dadurch erzielbare Kostenersparnis höher oder geringer ist als die vorstehend erwähnte Kostensteigerung. Insbesondere kann es daher aus individuellen betriebswirtschaftlichen Analysen heraus vorteilhaft sein, die recycelbare/recycelte Wärmeenergie anderweitig einzusetzen, nämlich so, dass ggf. auf maschineninterne Wärmetauscher verzichtet werden kann oder diese zumindest kleiner dimensionierbar sind.

Auch kann nach neuen Erkenntnissen der individuelle Betrieb der Blutbehandlungs-/Dialysemaschinen dazu führen, dass Blutbehandlungs-/Dialysemaschinen zufällig in gleichen Betriebsphasen, etwa in einer Desinfektionsphase mit hohem Energieverbrauch betrieben werden. Dadurch kommt es zu temporären Stromverbrauchsspitzen, die ggf. tariflich bedingt zu überhöhten Stromgebühren führen. Der Einsatz der ggf. recycelten Primärenergie sollte demnach so gestaltet und beispielsweise mittels eines speziellen Datenmanagementsystems so gesteuert werden, dass eine Koordination der bisher nur individuell betriebenen Blutbehandlungs-/Dialysemaschinen ermöglicht wird, um solche Verbrauchsspitzen zu vermeiden, zumindest aber zu reduzieren.

Gemäß einem ersten, ggf. unabhängig zu beanspruchenden Aspekt der vorliegenden Erfindung ist die Blutbehandlungs-/Dialyseanlage in ihrem stationären Abschnitt mit einer (zentralen) Wasser-Aufbereitungseinrichtung vorzugsweise der Umkehrosmosebauart ausgerüstet, die mit frischem (Leitungs-) Wasser bzw. Speisewasser versorgt wird und an deren Ausgang wenigstens eine Wasserzuführleitung bzw. Fluid-Zuführleitung für (nicht stationäre) Blutbehandlungs-/Dialysemaschinen angeschlossen ist, die einen Abschnitt mit einer Anzahl von Zweiganschlüssen hat. An diese Zweiganschlüsse sind in selektiver Weise Blutbehandlungs-/Dialysemaschinen (auch Maschinen für Akutdialyse, Maschinen für Apherese, usw.) vorzugsweise der mobilen Bauart fluidgekoppelt bzw. fluidkoppelbar. Diese entsprechen hinsichtlich ihres Aufbaus im Wesentlichen aus dem Stand der Technik bekannten Maschinen, können jedoch gemäß der vorliegenden Erfindung ggf. ohne internen Wärmetauscher zur Energierückgewinnung ausgebildet sein. Ferner hat die Blutbehandlungs-/Dialyseanlage wenigstens eine Ablaufleitung, über die verbrauchte Reinigungs-/Dialysierflüssigkeit aus aktuell fluidgekoppelten Blutbehandlungs-/Dialysemaschinen gesammelt in einen (stationären) Abfluss oder einen Sammeltank entsorgbar ist.

Erfindungsgemäß ist ein Blutbehandlungs-/Dialysemaschinen-externer (stationärer/zentraler) erster Wärmetauscher (WT1) vorgesehen, dessen eine Seite an die Wasserzuführleitung bzw. Fluid-Zuführleitung zwischen der Wasser-Aufbereitungseinrichtung und einem in Strömungsrichtung hierzu nächst gelegenen Zweiganschluss bzw. dem Leitungsabschnitt mit den Zweiganschlüssen angeschlossen ist und dessen andere Seite an die Ablaufleitung und/oder den Sammelbehälter für die gesammelte verbrauchte Dialysierflüssigkeit angeschlossen ist.

In anderen Worten ausgedrückt, wird erfindungsgemäß unter Ausnutzung der in der verbrauchten Dialysierflüssigkeit enthaltenen Wärmeenergie unter Verwendung einer zentralen Heizung, insbesondere des ersten Wärmetauschers, das Wasser (Permeat/Dialysierflüssigkeit) im stationären Abschnitt/Bereich der Anlage erwärmt und über die Wasserzuführleitung, vorzugsweise in Form wenigstens einer Ringleitung, den Blutbehandlungs-/Dialysemaschinen zur Verfügung gestellt. Hierbei besteht grundsätzlich die Schwierigkeit, dass bei einem Erwärmen des Speisewassers für die (Umkehr-)Osmose das Risiko einer Verkeimung in der Wasser-Aufbereitungseinrichtung ansteigt. Da ferner für Osmosemembranen in der Regel Höchsttemperaturgrenzen gelten, ist die Höhe der Erwärmung des Speisewassers beschränkt und die Blutbehandlungs-/Dialysemaschinen müssten zwangsläufig elektrisch nachheizen. Dadurch würde die Nutzung der Energierückgewinnung eingeschränkt.

Indessen ist es erfindungsgemäß vorgesehen, das bereits aufbereitete Wasser (Permeat) nur stromab von der Aufbereitungseinrichtung vorzuwärmen, sodass eine Temperaturerhöhung der Osmosemembran vermieden wird.

Vorzugsweise bildet die Wasserzuführleitung, wie bereits vorstehend angedeutet, wenigstens eine Ringleitung, die stromab zu der Anzahl von Zweiganschlüssen nunmehr in Form zumindest einer Rücklaufleitung zur Wasser-Aufbereitungseinrichtung zurückgeführt ist. Dadurch geht ungenutztes, bereits vorerwärmtes Wasser (Permeat) nicht verloren, sondern wird über die Wasseraufbereitungseinrichtung oder unmittelbar stromab von dieser erneut dem ersten Wärmetauscher zur weiteren/erneuten Vorheizung zugeführt.

Zusätzlich kann es vorgesehen sein, dass eine weitere Temperatur-Übertragungs-/Rückgewinnungseinrichtung angeordnet ist, bestehend aus einem Blutbehandlungs-/Dialysemaschinen-externen zweiten Wärmetauscher, dessen eine Seite an die Wasserzuführleitung vorzugsweise zwischen der Wasser-Aufbereitungseinrichtung und dem ersten Wärmetauscher angeschlossen ist und dessen andere Seite an die Rücklaufleitung vorzugsweise zwischen der Wasser-Aufbereitungseinrichtung und einem in Strömungsrichtung hierzu entferntest gelegenen Zweiganschluss bzw. dem Leitungsabschnitt mit den Zweiganschlüssen angeschlossen ist. Alternativ oder zusätzlich hierzu kann eine Wärmepumpe vorgesehen sein, welche thermische Energie aus der Ablaufleitung und/oder der Rücklaufleitung aufnimmt und Nutzenergie auf die Wasserzuführleitung vorzugsweise zwischen der Wasser-Aufbereitungseinrichtung und dem in Strömungsrichtung hierzu nächst gelegenen Zweiganschluss bzw. dem Leitungsabschnitt mit den Zweiganschlüssen überträgt.

Mittels des zweiten Wärmetauschers findet also eine (weitere) Vorerwärmung unter Ausnutzung der Restenergie in dem ungenutzt durch die wenigstens eine Ringleitung geführten Wasser (Fluid/Flüssigkeit) statt, wodurch sich das zur Aufbereitungseinrichtung zurückgeführte Wasser (Flüssigkeit) abkühlt und somit ein Aufheizen der Aufbereitungseinrichtung (und der darin befindlichen Osmosemembran) vermieden wird. Die Wärmepumpe hat den Vorteil, dass auch die Restenergie in der verbrauchten Dialysierflüssigkeit noch recycelt wird, um in das Wasser (frische Dialysierflüssigkeit) der Wasserzuführleitung eingespeist zu werden. Dabei ist insbesondere die Wärmepumpe (mittels eines Datenmanagementsystems) elektronisch steuerbar, um so die Temperatur des Wassers (Flüssigkeit) in der Wasserzuführleitung auf einen geeigneten/gewünschten Wert zu erhöhen oder aber auch zu senken (in diesem Fall kann die Wärmepumpe in umgekehrter Weise als Kühleinrichtung betrieben werden).

Gemäß einem anderen, ggf. unabhängig zu beanspruchenden Aspekt der vorliegenden Erfindung ist die erfindungsgemäße Blutreinigungs-/Dialyseanlage mit einer zentralen (stationären) elektronischen Steuereinheit (Teil des Datenmanagementsystems) ausgerüstet, an die zumindest ein Temperatursensor angeschlossen ist, der die Ist-Temperatur in der Wasserzuführleitung zumindest zwischen dem ersten Wärmetauscher (WT1) und dem die Zweiganschlüsse aufweisenden Leitungsabschnitt misst, wobei die Steuereinheit dementsprechend einen Wärmeübertragungsregler im Bereich des ersten Wärmetauschers und/oder die Wärmepumpe für das Erreichen einer Soll-Temperatur ansteuert, wodurch die übertragene oder übertragbare Wärmemenge durch den ersten Wärmetauscher und/oder durch die Wärmepumpe einstellbar ist. Dadurch kann zunächst prinzipiell einem überhitzen des in der Ringleitung befindlichen Wassers vorgebeugt werden.

Vorzugsweise ist die zentrale elektronische Steuereinheit zudem an die aktuell fluidgekoppelten Blutbehandlungs-/Dialysemaschinen elektrisch angeschlossen oder anschließbar, um Informationen über deren aktuelle Betriebsphasen/Betriebsparameter zu erhalten und u.a. in Abhängigkeit hiervon den Wärmeübertragungsregler und/oder die Wärmepumpe anzusteuern. Durch diese Rückkopplung wird ein (vollautomatisches) Energiemanagement ermöglicht, wonach die Temperatur des in der WasserZuführleitung/Ringleitung befindlichen Wassers auf einen Wert eingestellt wird, der die individuell noch notwendige Energie für ein maschinen-internes Aufheizen des Wassers entsprechend der einzelnen Betriebsphasen der Dialysemaschinen auf ein Minimum reduziert. Vorteilhaft ist es hierfür, wenn die Soll-Temperatur in der Wasserzuführleitung/Ringleitung auf den kleinsten der von den aktuell angeschlossenen Dialysemaschinen geforderten Temperaturwerte eingestellt wird.

Insgesamt kann somit durch die zentrale Erhitzung und Steuerung die effektive Vorbereitungszeit der einzelnen Blutbehandlungs-/Dialysemaschinen reduziert werden. Die Temperatur der Dialysierflüssigkeit (des Fluids) kann dabei klinische Relevanz haben. So kann z.B. hyposensitiven Episoden bei Hämodialysepatienten durch kühle Dialysierflüssigkeit entgegengewirkt werden.

Gemäß einem anderen, ggf. unabhängig zu beanspruchenden Aspekt der vorliegenden Erfindung kann es vorgesehen sein, dass die elektronische Steuereinheit in den Betriebsablauf der aktuell fluidgekoppelten Blutbehandlungs-/Dialysemaschinen direkt/indirekt eingreift, um deren aktuelle Betriebsphasen so zu koordinieren, dass Betriebsphasen insbesondere mit hohem elektrischen Energieverbrauch zeitversetzt bei kleinstmöglicher zeitlicher Überlappung ausgeführt werden. Dadurch sind Stromverbrauchsspitzen vermeidbar/reduzierbar, sodass der Strom beispielsweise zu einem tariflich niedrigeren Preis eingekauft werden kann. In anderen Worten ausgedrückt wird beispielsweise in Dialysezentren häufig in Schichten gearbeitet. Der Zeitablauf ist daher ähnlich. Da die Maschinen nach einer extrakorporalen Blutbehandlung auch eine Desinfektion durchlaufen müssen, erhöht sich der Gesamt-Strombedarf in den Zentren nahezu zyklisch. Da allgemein bekannte Energieversorgungsunternehmen in der Regel neben dem reinen Stromverbrauch (in KWh) auch Gebühren in Abhängigkeit vom temporären Stromspitzenbedarf berechnen, ist es erfindungsgemäß vorgesehen, diesen zu minimieren. Mit der erfindungsgemäßen zentralen elektronischen Steuereinheit quasi als Datenknoten ist es nunmehr möglich, die programmgesteuerte Heizleistung im Voraus zu kommunizieren.

Die Desinfektion einer Blutbehandlungs-/Dialysemaschine läuft beispielsweise in den folgenden Phasen ab:
- Dialysierflüssigkeit ausspülen,
- Vorheizen,
- Desinfektionsmittel ansaugen,
- Desinfektionslösung aufheizen,
- heiße Desinfektionslösung zirkulieren,
- Desinfektionslösung ausspülen und Maschine abkühlen.
Die Heizleistung je Maschine für diesen Vorgang beträgt derzeit ca. 1500 - 2000 W.

Durch zeitversetztes Starten der Aufheizphase im Bereich von z.B. 15 Minuten kann der Spitzenstrombedarf um durchschnittlich ca. 20% gesenkt werden. Der hierdurch generierte zusätzliche Zeitbedarf kann problemlos durch die Wartezeit auf den (ggf. sich verspätenden) Patienten (entspricht einer durchschnittlichen Leerlaufzeit) in der Regel aufgefangen werden. Ein ebensolcher Zeitversatz kann sich auch beim Ausspülen der Desinfektionslösung ergeben. Somit reduziert sich auch die max. Wassermenge pro Zeiteinheit, welche insbesondere beim Ausspülen verbraucht wird.

In der behandlungsfreien Zeit rezirkuliert die Dialysierflüssigkeit in der einen oder den mehreren Ringleitung(en). Vergleichbar zum Kühlschrankprinzip würde eine Reduzierung der Temperatur zu einem verringerten Keimwachstum (Keimwachstumsrisiko) führen. Durch die optionale Einbeziehung der Wärmepumpe in Verbindung mit der erfindungsgemäßen zentralen elektronischen Steuerung kann dieses Risiko bei entsprechendem Kühlbetrieb der Wärmepumpe zentral reguliert und gesteuert werden. Ferner kann durch den Einsatz der optionalen Wärmepumpe im Kühlmodus die Temperatur der Blutreinigungs-/Dialysemaschine nach deren Desinfektion/Heißreinigung schnell und gezielt heruntergekühlt werden.

Durch die Bereitstellung des erfindungsgemäßen Datenmanagementsystems (einschließlich der zentralen Steuerung) können noch weitere Vorteile erzielt werden:
Bei einer zentralen Desinfektion findet optional eine sogenannte "Inline"-Heißreinigung statt, bei der nicht nur die zumindest eine Ringleitung (ggf. inklusive Aufbereitungsanlage) desinfiziert wird, sondern auch die Blutbehandlungsmaschinen. Über das erfindungsgemäße Datenmanagementsystem kann eine optimale Einbeziehung der aktuell angeschlossenen Maschinen in die Heißreinigung erreicht werden. Dies erfolgt durch zentrale (automatische) Ansteuerung der Blutbehandlungsmaschinen insbesondere nach Ablauf der Aufheizphase der Wasserversorgung. Die Blutbehandlungsmaschinen starten dann die Ansaugphase der zentralen Heißreinigung zeitversetzt, so dass die Wasser-Versorgungsleitungstemperatur quasi aufrechterhalten wird.

Ferner bewirkt das erfindungsgemäße Datenmanagementsystem eine erhöhte Betriebs und auch Patientensicherheit. Insbesondere kann mit Hilfe des erfindungsgemäßen Datenmanagementsystems (zentrale Steuerung) eine Kommunikation zwischen der Aufbereitungsanlage und den einzelnen Blutbehandlungsmaschinen stattfinden. Die Aufbereitungsanlage (Umkehrosmose) erhält des Weiteren Daten darüber, wie viele Blutbehandlungsmaschinen aktuell angeschlossen und somit wie viel Wasser hierfür bereitgestellt werden muss. Ergeben sich außerordentliche Abweichungen zwischen dem in das Leitungssystem geschickten und in die Aufbereitungsanlage zurückkommenden Wasser (Flüssigkeit), ist die Steuerung dafür angepasst, die Anlage automatisch abzuschalten und/oder ein Warnsignal zu geben.

Schließlich kann das Datenmanagementsystem dafür angepasst sein, eine Datensammlung zu entwickeln und zu pflegen (zu aktualisieren) und darauf basierend die Heizeinrichtung und/oder die Wärmepumpe auf entsprechende Betriebsparameter voreinzustellen.

### Figurenbeschreibung

Die Erfindung wird nachstehend anhand eines bevorzugten Ausführungsbeispiels unter Bezugnahme auf die begleitende Figur näher erläutert. Diese zeigt das Fluid-Schaltbild einer Dialyseanlage sowie die elektronische Steuereinrichtung zur Überwachung und Steuerung der fluidmechanischen Elemente der Dialyseanlage sowie zur zentralen Koordination der aktuell angeschlossenen Dialysemaschinen bzw. deren aktuelle Betriebsphasen.

Erfindungsgemäß lässt sich die Dialyseanlage gemäß der vorliegenden Erfindung prinzipiell in einen stationären Bereich und einen instationären/mobilen Bereich untergliedern. Der mobile Bereich betrifft im Wesentlichen Dialysemaschinen, die getrennt voneinander sowie wahlweise an den stationären Bereich anschließbar sind oder in Betrieb genommen werden können, um jeweils eine Dialysebehandlung an einem Patienten auszuführen. Der stationäre Bereich beinhaltet u.a. ein Rohrleitungssystem mit darin integrierten strömungstechnischen Funktionselementen (Ventile, Drosseln, Filtern, etc.) zur korrekten Versorgung der aktuell angeschlossenen/im Betriebszustand befindlichen Dialysemaschinen mit gereinigtem/vorbehandeltem Wasser sowie zur Entsorgung verbrauchter Fluide/Flüssigkeiten und eine zentrale Steuerung zur Bereitstellung des Wassers in einem gewünschten Zustand (gereinigt, temperiert, druckbeaufschlagt, etc.) mittels hierfür geeigneter Funktionselemente(, elektronische Steuereinheit, Temperatur/Drucksensoren, Wärmetauscher, Wärmepumpen, Druckpumpen, etc.)

Die Dialysemaschinen entsprechen hinsichtlich ihres jeweiligen Aufbaus und ihrer jeweiligen Funktion im Wesentlichen aus dem Stand der Technik bekannten Maschinen, können jedoch bei der vorliegenden Erfindung wärmetauscherlos oder mit nur klein dimensioniertem internem Wärmetauscher ausgebildet sein. Außerdem weisen die Dialysemaschinen jeweils eine elektronische I/0-Schnittstelle auf, über die Daten/Informationen bezüglich der jeweils aktuellen Betriebszustände/Betriebsphasen vom Dialysemaschinen-internen Rechner (CPU) abgerufen und Steuersignale zur externen Beeinflussung der aktuellen Betriebszustände/Betriebsphasen eingegeben werden können. Insofern richtet sich die nachfolgende Beschreibung hauptsächlich auf den stationären Bereich der erfindungsgemäßen Dialyseanlage.

Dieser Bereich hat zunächst eine Speisewasser-Zuführleitung 1 mit integriertem Filter, ggf. Wasserenthärter und ggf. Drossel, über die Speisewasser aus einer Speisewasserquelle (örtliche Wasserversorgung, Brunnen, etc.) vorgereinigt und auf einen bestimmten Druck reduziert einer Wasser-Aufbereitungsanlage 2, vorzugsweise Umkehrosmose-Filteranlage an deren Einlass zugeführt wird. In der Wasser-Aufbereitungsanlage 2 findet u. a. eine Wasserbehandlung statt, um das Speisewasser in ein dialyse-geeignetes Wasser aufzubereiten. Derartige WasserAufbereitungsanlagen sind hinlänglich aus dem Stand der Technik bekannt, sodass sich eine weitere Beschreibung an dieser Stelle erübrigt.

An den Auslass dieser Wasser-Aufbereitungsanlage 2 ist eine Zuführleitung 4 für dialyse-geeignetes Wasser (Fluid/Flüssigkeit) angeschlossen, die in einen Leitungsabschnitt 6 mit einer Anzahl von Anschlussstellen 10 mündet. An diese Anschlussstellen (Zapfstellen) 10 sind wahlweise Blutbehandlungs-/Dialysemaschinen 8 gemäß vorstehender Ausführung fluidtechnisch angeschlossen. Der Leitungsabschnitt 6 mit der Anzahl von Anschlussstellen 10 geht in Stromrichtung gesehen in eine Rückführleitung 12 über, die in die Wasser-Aufbereitungsanlage 2, insbesondere an deren Einlass zurückgeführt ist. Dadurch ergibt sich eine stationäre Ringleitung, in der permanent dialyse-geeignetes Wasser zirkuliert und immer wieder einem Reinigungsprozess in der Wasser-Aufbereitungsanlage 2 unterzogen wird. Alternativ hierzu ist es aber prinzipiell auch möglich, dass die Rückführleitung unmittelbar stromab zur Wasser-Aufbereitungsanlage in die Zuführleitung für dialyse-geeignetes Wasser (nachfolgend nur noch als Wasserzuführleitung bezeichnet) einmündet, wobei in diesem Fall gereinigtes Wasser aus der Aufbereitungsanlage nur noch in dem Umfang in die Ringleitung eingespeist wird, um aus den Anschlussstellen abgezapftes Volumen zu ersetzen. Schließlich kann in die Wasserzuführleitung und/oder die Rückführleitung eine Druckpumpe eingesetzt sein, um die Wasserzirkulation bei bestimmter Strömungsgeschwindigkeit in Gang zu halten.

Des Weiteren ist eine Sammelleitung 14 für verbrauchte Dialysierflüssigkeit vorzugsweise mit einer Anzahl von Anschlussstellen vorgesehen, an die die ausgewählten Dialysemaschinen 8 angeschlossen sind, um verbrauchte Dialysierflüssigkeit über die Sammelleitung 14 abzuführen und in einem Abfluss oder Behälter zu entsorgen. Diese Sammelleitung 14 wird daher nachstehend auch als Abwasserleitung oder Ablaufleitung bezeichnet.

In die Wasserzuführleitung 4 zwischen der Wasser-Aufbereitungsanlage 2 und dem Leitungsabschnitt 6 mit der Anzahl von Anschlussstellen 10 ist ein erster stationärer Wärmetauscher WT1 an dessen einer Seite WT1/1 zwischengeschaltet, dessen andere Seite WT1/2 in die Ablaufleitung 14 zwischengeschaltet ist. Auf diese Weise wird dem in der Ablaufleitung 14 abfließenden Fluid Wärme entzogen und auf das in der Wasserzuführleitung 4 strömende Wasser zugeführt und dieses dadurch erwärmt.

Vorzugsweise ist der erste stationäre Wärmetauscher WT1 bezüglich seiner Wärmeübertragungsleistung elektronisch/elektrisch regelbar. Zu diesem Zweck ist der erste stationäre Wärmetauscher WT1 bzw. dessen elektrische Regeleinrichtung RE an eine zentrale elektronische Steuereinheit (CPU) CT1 elektrisch angeschlossen. Des Weiteren sind eine Anzahl von Temperatursensoren T1-T4 an die elektronische Regeleinheit CT1 angeschlossen, welche in Strömungsrichtung beabstandet an der Wasserzuführleitung 4 angeordnet sind, derart, dass zumindest ein Temperatursensor stromauf und stromab zum ersten Wärmetauscher WT1 platziert ist. Vorzugsweise sind zwei Temperatursensoren T1, T4 stromab zum Wärmetauscher WT1 sowie in Stromrichtung beabstandet angeordnet.

Zwischen dem ersten stationären Wärmetauscher WT1 und der Wasser-Aufbereitungsanlage 2 ist ein zweiter stationärer Wärmetauscher WT2 an dessen einer Seite WT2/1 in die Wasserzuführleitung 4 zwischengeschaltet, dessen andere Seite WT2/2 in die Rückführleitung 12 zwischengeschaltet ist. Dabei befindet sich einer der Temperaturfühler T2 an der Wasserzuführleitung 4 zwischen den beiden Wärmetauschern WT1, WT2 und ein weiterer Temperaturfühler T3 an der Wasserzuführleitung 4 zwischen dem zweiten stationären Wärmetauscher WT2 und der Wasser-Aufbereitungsanlage 2. Auf diese Weise erhält die elektronische Steuereinheit CT1 Informationen über den Temperaturverlauf längs der Wasserzuführleitung 4 ausgehend von der Aufbereitungsanlage 2 bis zum Leitungsabschnitt 6 mit den Zweiganschlussstellen 10. Ferner ist die zentrale elektronische Steuereinheit CT1 an die aktuell an die Ringleitung fluid-angeschlossenen Dialysemaschinen 8 an deren jeweiligen I/0-Schnittstellen elektrisch angeschlossen, um Informationen über deren aktuelle Betriebszustände/Betriebsphasen zu erhalten und regelnd in deren aktuelle Betriebszustände/Betriebsphasen nach bestimmten Kriterien gemäß nachfolgender Beschreibung ggf. einzugreifen.

Schließlich ist eine stationäre Wärmepumpe WP vorgesehen, welche thermische (Rest) Energie aus der Ablaufleitung 14 und/oder der Rücklaufleitung 12 aufnimmt und Nutzenergie auf die Wasserzuführleitung 4 zwischen der Wasser-Aufbereitungseinrichtung 2 und dem die Zweiganschlüsse 10 aufweisenden Leitungsabschnitt 6 überträgt. Im Konkreten nimmt die Wärmepumpe WP noch verbleibende Wärmeenergie aus dem in der Ablaufleitung 14 stromab zum ersten Wärmetauscher WT1 abfließenden Fluid auf und gibt Wärmeenergie in das in der Wasserzuführleitung 4 stromab zum zweiten Wärmetauscher WT2 fließenden Wassers vorzugsweise im Bereich des ersten Wärmetauschers WT1 ab.

Die Funktionsweise der erfindungsgemäßen Dialyseanlage mit dem vorstehenden Aufbau lässt sich wie Folgt beschreiben:
Grundsätzlich wird in der Dialyseanlage der vorliegenden Erfindung Speisewasser in der Wasser-Aufbereitungsanlage 2 gereinigt, sodass es eine für die nachfolgende Blutreinigungs-/Dialysebehandlung geeignete Qualität erlangt. Anschließend wird das aufbereitete Wasser in die stromabwärtige Ringleitung geführt, in der es von der Wasserzuführleitung 4 und dem die Zweiganschlüsse 10 aufweisenden Leitungsabschnitt 6 in die Rückführleitung 12 gelangt und von dort wieder zur Wasser-Aufbereitungsanlage 2 gemäß einem Wasserkreislauf zurückgeleitet wird.

Mit Durchströmen des zweiten Wärmetauschers WT2 unmittelbar stromab zur Wasser-Aufbereitungsanlage 2 erfährt das Wasser in der Wasserzuführleitung 4 zunächst eine Vorerwärmung, indem Wärmeenergie der Rückführleitung 12 entzogen und in die Wasserzuführleitung 4 übertragen wird. Dadurch kommt das zur Aufbereitungsanlage 2 rückgeführte Wasser kalt dort an, sodass eine Temperaturerhöhung beispielsweise einer Osmosemembran in der Aufbereitungsanlage 2 vermieden wird.

Anschließend durchströmt das Wasser in der Wasserzuführleitung 4 den ersten Wärmetauscher WT1, der nunmehr in geregelter Weise Wärmeenergie aus der die verbrauchte Dialysierflüssigkeit (Fluid) fördernden Ablaufleitung 14 in die Wasserzuführleitung 4 überträgt. Zusätzlich wird optional die Wärmepumpe WP betrieben, welche die vom ersten Wärmetauscher WT1 nicht genutzte Restenergie in der verbrauchten Dialysierflüssigkeit der Ablaufleitung 14 entzieht und der zum ersten Wärmetauscher WT1 stromabwärtigen Abschnitt der Wasserzuführleitung 4 zuführt. Alternativ ist es aber auch möglich, die Wärmepumpe WP umgekehrt zu betreiben, um das Wasser in der Ringleitung zu kühlen, beispielsweise im Fall eines Desinfektionsvorgangs in einer Dialysemaschine 8, worauf die überhitzte Dialysemaschine 8 möglichst schnell auf Behandlungstemperatur abgekühlt werden muss.

Über die Temperaturfühler T1-T4 stromauf zum zweiten Wärmetauscher WT2, zwischen dem ersten und zweiten Wärmetauscher sowie stromab zum zweiten Wärmetauscher WT2 lässt sich der Temperaturverlauf längs der Wasserzuführleitung 4 messen, wobei die Messergebnisse der zentralen Steuereinheit CT1 zugeführt werden. Diese erhält auch Informationen von den aktuell angeschlossenen Dialysemaschinen 8 insbesondere darüber, in welcher aktuellen Betriebsphase sich die einzelne Dialysemaschine 8 befindet und welche Temperatur das zugeführte Wasser entsprechend haben muss.

Ausgehend hiervon bestimmt die zentrale Steuereinheit CT1 den jeweils niedrigsten geforderten Temperaturwert als Soll-Wert und reguliert die Wassertemperatur über den ersten Wärmetauscher WT1 und/oder die Wärmepumpe WP auf diesen, seitens aller aktuell angeschlossenen Dialysemaschinen 8 geforderten niedrigsten Wert. Durch die zentrale Erwärmung und Steuerung wird somit die effektive Vorbereitungszeit der einzelnen Dialysemaschinen 8 reduziert. Darüber hinaus hat die zentrale Steuereinheit CT1 gemäß der Erfindung noch weiterreichende Steuermöglichkeiten:
In Dialysezentren wird in der Regel im Schichtbetrieb gearbeitet. Der Zeitablauf ist also ähnlich und wiederholt sich kontinuierlich. Da die Dialysemaschinen 8 nach einer Dialysebehandlung grundsätzlich eine Desinfektionsphase durchlaufen müssen, erhöht sich der Gesamt-Strombedarf in Zentren in zyklischer Weise überproportional insbesondere dann, wenn mehrere Dialysemaschinen 8 gleichzeitig die Desinfektionsphase durchlaufen. Da Energieversorger neben dem reinen Stromverbrauch auch Gebühren in Abhängigkeit vom Spitzenstrombedarf berechnen, ist es sinnvoll, derartige Stromspitzen möglichst zu vermeiden oder zu minimieren.

Die Energie intensive Desinfektion einer Dialysemaschine läuft im Allgemeinen in den folgenden Schritten ab:
- Dialysierflüssigkeit ausspülen,
- Vorheizen,
- Desinfektionsmittel ansaugen,
- Desinfektionsmittel-Wasser-Lösung (oder einfach Desinfektionslösung) aufheizen,
- heiße Lösung zirkulieren,
- heiße Lösung ausspülen,
- Dialysemaschine abkühlen.
Die Heizleistung je Maschine beträgt dabei zwischen 1500 bis 2000 W.

Die zentrale Steuereinheit CT1 erhält, wie vorstehend bereits angedeutet wurde, Informationen über die aktuellen Betriebsphasen der angeschlossenen Dialysemaschinen 8 und ist dafür angepasst, in die Dialysemaschinen-interne Steuerung einzugreifen, derart, dass durch zeitversetztes Starten der vorstehend genannten Aufheizphase (beispielsweise im Bereich von ca. 15 Minuten) der Spitzenstrombedarf gesenkt werden kann (um ca. 20% gegenüber gleichgeschalteten Dialysemaschinen). Basierend auf der vorstehenden Kommunikation zwischen zentraler Steuereinheit CT1 und den jeweils angeschlossenen Dialysemaschinen 8 ergibt sich bei entsprechenden zeitversetzten Aufheizphasen auch ein ebensolcher Zeitversatz für den jeweiligen Ausspülschritt, wodurch sich auch die max. Wassermenge reduziert, die ja beim Ausspülen genutzt wird.

In der dialysefreien Zeit bleibt die Rezirkulation des Wassers in der Ringleitung bestehen. Ähnlich wie bei dem Kühlschrankprinzip kann eine Reduzierung der Temperatur zu einer verringerten Keimwachstum führen. Durch die Einbeziehung der Wärmepumpe WP in die Dialyseanlage gemäß der Erfindung in Verbindung mit der zentralen Steuereinheit CT1 kann die Wassertemperatur in der Ringleitung bei nicht in Betrieb befindlichen Dialysemaschinen auf einen geeigneten niedrigen Wert abgesenkt werden. Des Weiteren kann, wie vorstehend bereits angedeutet wurde, die Wärmepumpe WP dazu genutzt werden, die angeschlossenen Dialysemaschinen 8 nach der Desinfektionsphase schnell und gezielt abzukühlen.

Durch die Kommunikation zwischen stationärer Wasserversorgung (einschließlich Wasser-Aufbereitungsanlage) und Dialysetechnologie wird eine Schnittstelle geschaffen, welche neben den vorstehend genannten Effekten noch zu weiteren Nutzervorteilen führt:
- Bei der zentralen Desinfektion kann eine Inline-Heißreinigung stattfinden. Hierbei werden nicht nur die Ringleitung (ggf. inklusiver der Wasser-Aufbereitungsanlage) desinfiziert sondern auch die Dialysemaschinen eingebunden. Über die zentrale Steuereinheit kann eine optimale Einbeziehung der Dialysemaschinen in die Heißreinigung eingestellt werden. Dies geschieht durch eine zentrale Ansteuerung der jeweiligen Dialysemaschinen nach Ablauf der Aufheizphase der Wasserversorgung. Die Dialysemaschinen werden in diesem Fall dazu veranlasst, die Ansaugphase der zentralen Heißreinigung zeitversetzt zu starten, sodass die Temperatur in der Versorgungsleitung im Wesentlichen aufrechterhalten werden kann. Die Synchronisation dieses Vorgangs erfolgt somit zentral durch die Steuereinheit im Gegensatz zu der aktuell gängigen Praxis, wonach jede Maschine einzeln programmiert werden muss.
- Die Einbindung der zentralen Steuereinheit in die Synchronisation/Koordination der Betriebsphasensteuerung hat eine erhöhte Sicherheit zur Folge. In der Vergangenheit sind zahlreiche Vor-/Unfälle bekannt geworden, wonach sich Schlauchverbindungen zwischen den Zweiganschlüssen und den Dialysemaschinen selbständig gelöst haben oder beschädigt wurden, was zu hohen Wasserschäden im Dialysezentrum führte. Mit Hilfe der zentralen Steuereinheit kann jedoch eine Kommunikation zwischen den einzelnen Dialysemaschinen und der stationären Wasserversorgung stattfinden. Die Wasserversorgung bzw. deren Steuerung erhält Daten, wie viele Dialysemaschinen beispielsweise in die Heißreinigung eingebunden sind und wie viel Wasser hierfür bereitgestellt werden muss. Stimmt das Verhältnis zwischen dem in die Ringleitung geschickte und in die Wasser-Aufbereitungsanlage zurückkommende Wasser nicht, kann die zentrale Steuereinheit auf das Vorhandensein eines Lecks schließen und die Anlage z.B. automatisch abschalten. Kostenintensive Schäden sind somit vermeidbar.

Zusammenfassend wird eine Blutbehandlungs-/Dialyseanlage offenbart mit einer Wasser-Aufbereitungseinrichtung vorzugsweise der Umkehrosmosebauart, an deren Ausgang eine Wasserzuführleitung angeschlossen ist, die eine Anzahl von Zweiganschlüssen hat, an die in selektiver Weise Blutbehandlungs-/Dialysemaschinen fluidgekoppelt sind und mit einer Ablaufleitung, über die verbrauchte Flüssigkeit (Dialysierflüssigkeit) aus fluidgekoppelten Blutbehandlungs-/Dialysemaschinen entsorgbar ist. Erfindungsgemäß ist wenigstens ein Maschinen-externer Wärmetauscher vorgesehen, dessen eine Seite an die Wasserzuführleitung unmittelbar stromauf zu den Zweiganschlüssen und dessen andere Seite an die Ablaufleitung angeschlossen.

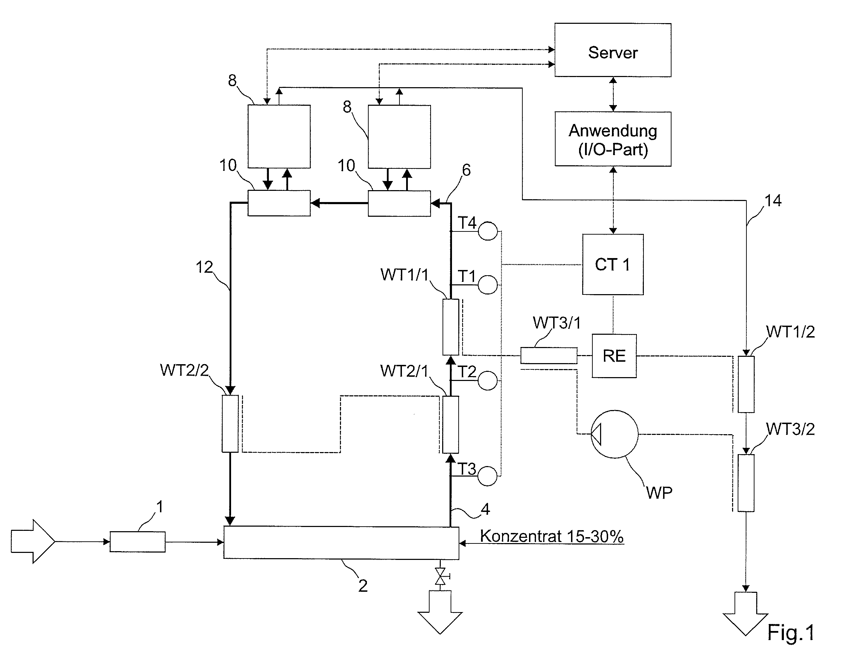

## Patentansprüche

1. Extrakorporale Blutreinigungsanlage mit
- einem stationären Anlagebereich, unter anderem aufweisend:
eine Wasser-Aufbereitungseinrichtung (2) vorzugsweise der Umkehrosmosebauart, an deren Ausgang wenigstens eine Wasserzuführleitung (4) angeschlossen ist, die in zumindest einen Leitungsabschnitt (6) mit einer Anzahl von Zweiganschlüssen (10) mündet, und
wenigstens eine Ablaufleitung (14), über die verbrauchte Blutreinigungs-/oder Dialysierflüssigkeit entsorgbar ist, sowie mit
- einem instationären Anlagebereich, aufweisend:
eine Anzahl von Blutreinigungsmaschinen (8) die in selektiver Weise mit den Zweiganschlüssen (10) und der wenigstens einen Ablaufleitung (14) fluidgekoppelt sind,
**gekennzeichnet durch**
- eine zentrale elektronische Steuereinheit (CT1), welche an die aktuell fluidgekoppelten Blutreinigungsmaschinen (8) elektrisch angeschlossen ist, um Informationen zumindest über deren aktuellen Betriebsphasen zu erhalten und welche in Abhängigkeit hiervon eine an die wenigstens eine Wasserzuführleitung (4) angeschlossene stationäre Heizeinrichtung so ansteuert, dass sich eine Soll-Temperatur in der wenigstens einen Wasserzuführleitung (4) auf den kleinsten der von den aktuell angeschlossenen Blutreinigungsmaschinen (8) entsprechend deren jeweiligen Betriebsphasen geforderten Temperaturwerte einstellt.

2. Extrakorporale Blutreinigungsanlage nach Anspruch 1, **dadurch gekennzeichnet, dass**
eine Blutbehandlungsmaschinen-externe, stationäre Heizeinrichtung mit einem ersten Wärmetauscher (WT1), dessen eine Seite (WT1/1) an die wenigstens eine Wasserzuführleitung (4) zwischen der Wasser-Aufbereitungseinrichtung (2) und dem die Zweiganschlüsse (10) aufweisenden Leitungsabschnitt (6) angeschlossen ist und dessen andere Seite (WT1/2) an die wenigstens eine Ablaufleitung (14) angeschlossen ist, vorgesehen ist, und
an die zentrale elektronische Steuereinheit (CT1) zumindest ein Temperatursensor (T1-T4) angeschlossen ist, der die Ist-Temperatur in der Wasserzuführleitung (4) zumindest zwischen dem ersten Wärmetauscher (WT1) und dem die Zweiganschlüsse aufweisenden Leitungsabschnitt (6) misst und dementsprechend die stationäre Heizeinrichtung für das Erreichen der Soll-Temperatur ansteuert.

3. Extrakorporale Blutreinigungsanlage nach Anspruch 1 oder 2, **dadurch gekennzeichnet, dass** die elektronische Steuereinheit (CT1) in den Betriebsablauf der aktuell fluidgekoppelten Blutbehandlungsmaschinen (8) eingreift, um deren aktuelle Betriebsphasen so zu koordinieren, dass Betriebsphasen mit hohem elektrischen Energieverbrauch zeitversetzt bei kleinstmöglicher zeitlicher Überlappung ausgeführt werden.

4. Extrakorporale Blutreinigungsanlage nach einem der vorstehenden Ansprüche, **dadurch gekennzeichnet, dass** die elektronische Steuereinheit (CT1) dazu konfiguriert ist, eine programmgesteuerte Heizleistung im Voraus zu kommunizieren und dadurch einen temporären Stromspitzenbedarf zu minimieren.

5. Extrakorporale Blutreinigungsanlage nach einem der vorstehenden Ansprüche, **dadurch gekennzeichnet, dass** die aktuell fluidgekoppelten Blutreinigungsmaschinen (8) mittels eines Datenmanagementsystems koordiniert gesteuert und dadurch Verbrauchsspitzen vermieden oder reduziert werden.

6. Extrakorporale Blutreinigungsanlage nach einem der vorstehenden Ansprüche, **dadurch gekennzeichnet, dass** die Wasserzuführleitung (4) zumindest eine Ringleitung bildet, die stromab zu dem die Anzahl von Zweiganschlüssen aufweisenden Leitungsabschnitt (6) in Form wenigstens einer Rücklaufleitung (12) zur Wasser-Aufbereitungseinrichtung (2) zurückgeführt ist.

7. Extrakorporale Blutreinigungsanlage nach Anspruch 2, **dadurch gekennzeichnet, dass** die stationäre Heizeinrichtung eine weitere Temperaturübertragungseinrichtung aufweist bestehend aus
- einem Blutbehandlungsmaschinen-externen zweiten Wärmetauscher (WT2) und/ oder einer Wärmepumpe (WP).

8. Extrakorporale Blutbehandlungsanlage nach Anspruch 7, **dadurch gekennzeichnet, dass** die eine Seite (WT2/1) des zweiten Wärmetauschers (WT2) an die wenigstens eine Wasserzuführleitung (4) vorzugsweise zwischen der Wasser-Aufbereitungseinrichtung (2) und dem ersten Wärmetauscher (WT1) angeschlossen ist und die andere Seite (WT2/2) des zweiten Wärmetauschers (WT2) an die wenigstens eine Rücklaufleitung (12) vorzugsweise zwischen der Wasser-Aufbereitungseinrichtung (2) und dem die Zweiganschlüsse aufweisenden Leitungsabschnitt (6) angeschlossen ist, wobei
- die Wärmepumpe (WP)bei deren Anordnung dafür angepasst ist, thermische Energie aus der wenigstens einen Ablaufleitung (14) und/oder der wenigstens einen Rücklaufleitung (12) aufzunehmen und Nutzenergie auf die Wasserzuführleitung (4) vorzugsweise zwischen der Wasser-Aufbereitungseinrichtung (2) und dem die Zweiganschlüsse aufweisenden Leitungsabschnitt (6) zu übertragen.

9. Extrakorporale Blutreinigungsanlage nach Anspruch 7 oder 8, **dadurch gekennzeichnet, dass** im Fall einer Anordnung der Wärmepumpe (WP) diese stromab zum ersten Wärmetauscher (WT1) an die wenigstens eine Ablaufleitung (14) angeschlossen ist und die Nutzenergie an die eine Seite (WT1/1) des ersten Wärmetauschers (WT1) abgibt.

10. Extrakorporale Blutreinigungsanlage nach Anspruch 7 oder 8, **dadurch gekennzeichnet, dass** im Fall einer Anordnung der Wärmepumpe (WP) diese jeweils unmittelbar stromab zum ersten Wärmetauscher (WT1) an die wenigstens eine Ablaufleitung (14) und die Wasserzuführleitung (4) angeschlossen ist.

11. Extrakorporale Blutreinigungsanlage nach einem der vorstehenden Ansprüche, **dadurch gekennzeichnet, dass** die zentrale elektronische Steuereinheit (CT1) dafür angepasst ist, Informationen bezüglich der entnommenen Wassermenge zu erhalten und diese mit einem vorzugsweise auf Basis der aktuellen Betriebsphasen abgeleiteten oder berechneten Bedarf der angeschlossenen Blutbehandlungsmaschinen zu bilanzieren, um hieraus das Vorliegen und vorzugsweise das Ausmaß einer Leckage zu bestimmen.

12. Extrakorporale Blutreinigungsanlage nach einem der vorstehenden Ansprüche, **dadurch gekennzeichnet, dass** die zentrale elektronische Steuereinheit (CT1) dafür angepasst ist, in der blutbehandlungsfreien Betriebsphase aller angeschlossenen Blutbehandlungsmaschinen die Temperatur der Flüssigkeit auf einen Wert oder in einen Wertebereich zu regeln, der zur Verringerung des Keimwachstums zumindest in der Wasserzuführleitung (4) beiträgt.

13. Extrakorporale Blutreinigungsanlage nach einem der vorstehenden Ansprüche, **dadurch gekennzeichnet, dass** die zentrale elektronische Steuereinheit (CT1) die Informationen, vorzugsweise bezüglich einer gewünschten DialysierflüssigkeitsTemperatur, -Fluss und/oder -Menge, einer aktuellen Desinfektionsphase voraussichtlichen Stillstandszeit, etc., sammelt und basierend hierauf eine entsprechend sich verändernde oder veränderbare Voreinstellung für die Soll-Temperatur vornimmt.

14. Extrakorporale Blutreinigungsanlage nach einem der vorstehenden Ansprüche, **dadurch gekennzeichnet, dass** diese als Dialyseanlage ausgebildet ist, wobei die extrakorporalen Blutbehandlungsmaschinen Dialysemaschinen der mobilen Bauart sind.

## Claims

1. An extracorporeal blood treatment system comprising
- a stationary system area including among other components:
a water treatment unit (2), preferably of the reverse osmosis type, whose outlet has connected thereto at least one water supply line (4) that opens into at least one line section (6) provided with a number of branch connections (10) and
at least one drain line (14) through which exhausted blood treatment/dialysis fluid can be discharged, and further comprising
- a non-stationary system area including:
a number of blood treatment machines (8) fluidly coupled to the branch connections (10) and the at least one drain line (14) in a selective manner, **characterized by**
- a central electronic control unit (CT1) which is electrically connected to the blood treatment machines (8) fluidly coupled at the time in question so as to obtain information on at least the current operating phases of these machines and which, in accordance with this information, controls a stationary heating unit that is connected to the at least one water supply line (4), such that a target temperature in the at least one water supply line (4) is adjusted to the smallest temperature value of the temperature values which the blood treatment machines (8) fluidly coupled at the time in question demand in accordance with their respective operating phases.

2. The extracorporeal blood treatment system according to claim 1, **characterized in that**
a stationary heating unit disposed externally to the blood treatment machine and comprising a first heat exchanger (WT1) having one side (WT1/1) thereof connected to the at least one water supply line (4) between the water treatment unit (2) and the line section (6) including the branch connections (10), and having the other side (WT1/2) thereof connected to the at least one drain line (14),
the central electronic control unit (CT1) has connected thereto at least one temperature sensor (T1-T4), which measures the actual temperature in the water supply line (4) at least between the first heat exchanger (WT1) and the line section (6) including the branch connections and which controls in accordance therewith the stationary heating unit so as to accomplish the target temperature.

3. The extracorporeal blood treatment system according to claim 1 or 2, **characterized in that**
the electronic control unit (CT1) intervenes in the operating
sequence of the blood treatment machines (8) fluidly coupled at the time in question, so as to coordinate their current operating phases such that operating phases with high electric power consumption are performed with a time shift such that they temporally overlap one another as little as possible.

4. The extracorporeal blood treatment system according to one of the preceding claims, **characterized in that**
the electronic control unit (CT1) is configured to communicate a program-controlled heating power in advance and to thereby minimize a temporary power-peak demand.

5. The extracorporeal blood treatment system according to one of the preceding claims, **characterized in that**
the blood treatment machines (8) fluidly coupled at the time in question are coordinatedly controlled by means of a data management system and consumption peaks can thereby be avoided or reduced.

6. The extracorporeal blood treatment system according to one of the preceding claims, **characterized in that**
the water supply line (4) defines at least one ring line, which, downstream of the line section (6) including the branch connections, is returned to the water treatment unit (2), said ring line being there configured as
at least one feedback line (12).

7. The extracorporeal blood treatment system according to one of the claim 2, **characterized in that**
the stationary heating unit comprises a further temperature transfer unit consisting of
- a second heat exchanger (WT2) disposed externally to the blood treatment machines and/or of a heat pump (WP).

8. The extracorporeal blood treatment system according to claim 7, **characterized in that**
one side (WT2/1) of the second heat exchanger (WT2) is connected to the at least one water supply line (4), preferably between the water treatment unit (2) and the first heat exchanger (WT1), and the other side (WT2/2) of the second heat exchanger (WT2) is connected to the at least one feedback line (12), preferably between the water treatment unit (2) and the line section (6) including the branch connections, wherein
- the heat pump (WP) is arranged such that it is adapted to take up thermal energy from the at least one drain line (14) and/or the at least one feedback line (12) and transfer useful energy to the water supply line (4), preferably between the water treatment unit (2) and the line section (6) including the branch connections.

9. The extracorporeal blood treatment system according to claim 7 or 8, **characterized in that,**
if a heat pump (WP) is arranged, said heat pump (WP) is connected to the at least one drain line (14) downstream of the first heat exchanger (WT1) and transfers the useful energy to one side (WT1/1) of the first heat exchanger (WT1).

10. The extracorporeal blood treatment system according to claim 7 or 8, **characterized in that,**
if a heat pump (WP) is arranged, said heat pump (WP) is connected to the at least one drain line (14) and the water supply line (4) immediately downstream of the first heat exchanger (WT1).

11. The extracorporeal blood treatment system according to one of the preceding claims, **characterized in that**
the central electronic control unit (CT1) is adapted to receive information with respect to the amount of water taken and to offset the latter against an amount required by the connected blood treatment machines and preferably derived from or calculated on the basis of the current operating phases, so as to determine therefrom the existence and preferably the degree of leakage.

12. The extracorporeal blood treatment system according to one of the preceding claims, **characterized in that**
the central electronic control unit (CT1) is adapted to control, in the blood treatment-free operating phase of all connected blood treatment machines, the temperature of the fluid such that it lies at a value or in a range of values contributing to a reduction of microbial growth at least in the water supply line (4).

13. The extracorporeal blood treatment system according to one of the preceding claims, **characterized in that**
the central electronic control unit (CT1) collects information, preferably information with respect to a desired temperature, flow and/or quantity of the dialysis fluid, a current disinfection phase, a presumable downtime, etc., and executes on the basis of such information an adequately varying or variable preadjustment for the target temperature.

14. The extracorporeal blood treatment system according to one of the preceding claims, **characterized in that**
it is configured as a dialysis system, the extracorporeal blood treatment machines being dialysis machines of mobile construction.

## Revendications

1. Installation d'épuration extracorporelle du sang avec
- une zone d'installation stationnaire, présentant entre autres :
un dispositif de traitement d'eau (2) de préférence du type de construction à osmose inverse, à la sortie duquel est raccordée au moins une conduite d'acheminement de l'eau (4) qui débouche dans au moins un segment de conduite (6) avec un certain nombre de raccords de dérivation (10), et
au moins une conduite d'évacuation (14) par laquelle du liquide d'épuration du sang ou de dialyse peut être éliminé, ainsi que
- une zone d'installation non stationnaire, présentant :
un certain nombre de machines d'épuration du sang (8) qui sont en communication de fluide de façon sélective avec les raccords de dérivation (10) et l'au moins une conduite d'évacuation (14),
**caractérisée en ce que**
- une unité de commande électronique (CT1) centrale qui est raccordée électriquement aux machines d'épuration du sang (8) actuellement en communication de fluide pour obtenir des informations au moins sur leurs phases de fonctionnement effectives et qui commande en fonction de celles-ci un dispositif de chauffage stationnaire raccordé à l'au moins une conduite d'acheminement de l'eau (4) de telle sorte qu'une température prescrite dans l'au moins une conduite d'acheminement de l'eau (4) s'ajuste à la plus petite des valeurs de températures exigées par les machines d'épuration du sang (8) effectivement raccordées conformément à leurs phases de fonctionnement respectives.

2. Installation d'épuration extracorporelle du sang selon la revendication 1, **caractérisée en ce que**
est prévu un dispositif de chauffage stationnaire externe aux machines de traitement du sang avec un premier échangeur de chaleur (WT1) dont un côté (WT1/1) est raccordé à l'au moins une conduite d'acheminement de l'eau (4) entre le dispositif de traitement d'eau (2) et le segment de conduite (6) présentant les raccords de dérivation (10) et dont l'autre côté (WT1/2) est raccordé à l'au moins une conduite d'évacuation (14), et
au niveau de l'unité de commande électronique (CT1) centrale est raccordé au moins un capteur de température (T1-T4) qui mesure la température réelle dans la conduite d'acheminement de l'eau (4) au moins entre le premier échangeur de chaleur (WT1) et le segment de conduite (6) présentant les raccords de dérivation et commande de façon correspondante le dispositif de chauffage stationnaire pour atteindre la température prescrite.

3. Installation d'épuration extracorporelle du sang selon la revendication 1 ou 2, **caractérisée en ce que** l'unité de commande électronique (CT1) intervient dans le déroulement du fonctionnement des machines de traitement du sang (8) effectivement en communication de fluide pour coordonner leurs phases de fonctionnement effectives de telle sorte que des phases de fonctionnement avec une consommation d'énergie électrique élevée soient exécutées en différé avec un chevauchement temporel le plus faible possible.

4. Installation extracorporel d'épuration du sang selon l'une des revendications précédentes, **caractérisée en ce que** l'unité de commande électronique (CT1) est configurée pour communiquer à l'avance une performance de chauffage commandée par un programme et minimiser ainsi un besoin temporaire de pic de courant.

5. Installation d'épuration extracorporelle du sang selon l'une des revendications précédentes, **caractérisée en ce que** les machines d'épuration du sang (8) effectivement en communication de fluide sont commandées de façon coordonnée au moyen d'un système de gestion de données, des pics de consommations étant ainsi évités ou réduits.

6. Installation extracorporel d'épuration du sang selon l'une des revendications précédentes, **caractérisée en ce que** la conduite d'acheminement de l'eau (4) forme au moins une conduite annulaire qui est reconduit en aval du segment de conduite (6) présentant le nombre de raccords de dérivation, sous la forme d'au moins une conduite de retour (12) vers le dispositif de traitement d'eau (2).

7. Installation d'épuration extracorporelle du sang selon la revendication 2, **caractérisé en ce que** le dispositif de chauffage stationnaire présente un autre dispositif de transmission de température consistant en
- un deuxième échangeur de chaleur (WT2) externe aux machines d'épuration du sang et/ou une pompe à chaleur (WP).

8. Installation d'épuration extracorporelle du sang selon la revendication 7, **caractérisé en ce qu'**un côté (WT2/1) du deuxième échangeur de chaleur (WT2) est raccordé à l'au moins une conduite d'acheminement de l'eau (4), de préférence entre le dispositif de traitement d'eau (2) et le premier échangeur de chaleur (WT1), et **en ce que** l'autre côté (WT2/2) du deuxième échangeur de chaleur (WT2) est raccordé à l'au moins une conduite de retour (12), de préférence entre le dispositif de traitement d'eau (2) et le segment de conduite (6) présentant les raccords de dérivation, dans laquelle
- la pompe à chaleur (WP) est adaptée dans son agencement pour absorber de l'énergie thermique de l'au moins une conduite d'évacuation (14) et/ou de l'au moins une conduite de retour (12) et transmettre de l'énergie utile à la conduite d'acheminement d'eau (4), de préférence entre le dispositif de traitement d'eau (2) et le segment de conduite (6) présentant les raccords de dérivation.

9. Installation d'épuration extracorporelle du sang selon la revendication 7 ou 8, **caractérisée en ce que**, dans le cas d'un agencement de la pompe à chaleur (WP), celle-ci est raccordée en aval par rapport au premier échangeur de chaleur (WT1) à l'au moins une conduite d'évacuation (14) et délivre l'énergie utile à un côté (WT1/1) du premier échangeur de chaleur (WT1).

10. Installation d'épuration extracorporelle du sang selon la revendication 7 ou 8, **caractérisée en ce que**, dans le cas d'un agencement de la pompe à chaleur (WP) celle-ci est raccordée directement en aval par rapport au premier échangeur de chaleur (WT1) respectivement à l'au moins une conduite d'évacuation (14) et à la conduite d'acheminement d'eau (4).

11. Installation d'épuration extracorporelle du sang selon l'une des revendications précédentes, **caractérisée en ce que** l'unité de commande électronique (CT1) centrale est adaptée pour obtenir des informations relatives à la quantité d'eau prélevée et pour les équilibrer par rapport à un besoin dérivé ou calculé de préférence sur la base des phases de fonctionnement effectives des machines de traitement du sang raccordées pour déterminer à partir de là, la présence et de préférence l'ampleur d'une fuite.

12. Installation d'épuration extracorporelle du sang selon l'une des revendications précédentes, **caractérisée en ce que** l'unité de commande électronique (CT1) centrale est adaptée pour régler, dans la phase de fonctionnement sans traitement sanguin de toutes les machines de traitement du sang raccordées, la température du liquide à une valeur ou dans une plage de valeurs qui contribue à réduire la croissance des germes au moins dans la conduite d'acheminement de l'eau (4).

13. Installation d'épuration extracorporelle du sang selon l'une des revendications précédentes, **caractérisée en ce que** l'unité de commande électronique (CT1) centrale récolte les informations, de préférence relatives à une température, un flux et/ou une quantité de liquide de dialyse souhaité(e), à un temps d'arrêt prévu d'une phase de désinfection effective, etc. et effectue sur la base de celles-ci, un préréglage se modifiant ou pouvant être modifié de façon correspondante pour la température prescrite.

14. Installation d'épuration extracorporelle du sang selon l'une des revendications précédentes, **caractérisée en ce que** celle-ci est réalisée sous la forme d'une installation de dialyse, dans laquelle les machines de traitement extracorporel du sang sont des machines de dialyse de type de construction mobile.
